(19) Europäisches Patentamt
European Patent Office
Office européen des brevets

(11) Veröffentlichungsnummer: **0 538 690 B1**

(12) # EUROPÄISCHE PATENTSCHRIFT

(45) Veröffentlichungstag der Patentschrift: **28.09.94**

(51) Int. Cl.5: **C07D 401/04**, C07D 401/14, A61K 31/47

(21) Anmeldenummer: **92117261.5**

(22) Anmeldetag: **09.10.92**

Die Akte enthält technische Angaben, die nach dem Eingang der Anmeldung eingereicht wurden und die nicht in dieser Patentschrift enthalten sind.

(54) **Aryl-Chinolyl-substituierte 1,4-Dihydropyridin-dicarbonsäurederivate, Verfahren zu ihrer Herstellung und ihre Verwendung in Arzneimitteln.**

(30) Priorität: **22.10.91 DE 4134760**

(43) Veröffentlichungstag der Anmeldung: **28.04.93 Patentblatt 93/17**

(45) Bekanntmachung des Hinweises auf die Patenterteilung: **28.09.94 Patentblatt 94/39**

(84) Benannte Vertragsstaaten: **AT BE CH DE DK ES FR GB GR IT LI LU NL PT SE**

(56) Entgegenhaltungen:
**EP-A- 0 288 758**
**DE-A- 2 117 571**
**DE-A- 2 210 667**
**DE-A- 4 011 105**
**US-A- 3 946 026**

(73) Patentinhaber: **BAYER AG**

**D-51368 Leverkusen (DE)**

(72) Erfinder: **Stoltefuss, Jürgen, D.I.**
**Parkstrasse 20**
**W-5657 Haan (DE)**
Erfinder: **Goldmann, Siegfried, Dr.**
**Am Osterholz 91**
**W-5600 Wuppertal 1 (DE)**
Erfinder: **Straub, Alexander, Dr.**
**Moospfad 30**
**W-5600 Wuppertal 1 (DE)**
Erfinder: **Gross, Rainer, Prof. Dr.**
**Platzhofstrasse 23**
**W-5600 Wuppertal 1 (DE)**
Erfinder: **Bechem, Martin, Dr.**
**Hans-Böckler-Strasse 102**
**W-5600 Wuppertal 1 (DE)**
Erfinder: **Hebisch, Siegbert, Dr.**
**Richard-Seel-Weg 11**
**W-5600 Wuppertal 1 (DE)**
Erfinder: **Hütter, Joachim, Dr.**
**Teschen-Sudberger-Strasse 13**
**W-5600 Wuppertal 12 (DE)**
Erfinder: **Rounding, Howard-Paul, Dr.**
**Pahlkestrasse 15**
**W-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue Aryl-Chinolyl-substituierte 1,4-Dihydropyridin-dicarbonsäurederivate, Verfahren zu ihrer Herstellung und ihre Verwendung in Arzneimitteln, insbesondere zur Herstellung von Mitteln zur Behandlung von Herz-Kreislauferkrankungen.

Es ist bereits bekannt, daß 1,4-Dihydropyridine gefäßerweiternde Eigenschaften besitzen und als Coronarmittel und Antihypertensiva verwendet werden können [vgl. Brit. Patent 1 430 926]. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können [vgl. Fleckenstein, Ann. Rev. Pharmacol. Toxicol., 17, 149-166 (1977)].

Außerdem ist bekannt, daß Dihydropyridine mit ähnlichen Strukturen als Kreislauf- und Coronarmittel eingesetzt werden können. In DE-A-4011105 werden z. B. 4-Chinolyl-1,4-dihydropyridinderivate beschrieben, die sich nur in 3-Position des Dihydropyridinringes von den erfindungsgemäßen Verbindungen unterscheiden. Diese strukturell naheliegenden Verbindungen aus dem Stand der Technik zeigen jedoch eine positiv inotrope Wirkung während die erfindunsgemäßen 4-Chinolyl-dihydropyridine negativ inotrope Eigenschaften haben. Weitere 1,4-Dihydropyridine mit 3,5-Diester und/oder -Acylgruppierungen und auch 4-Chinolyl-1,4-dihydropyridine mit anderen Strukturen sind bereits bekannt z. B. in DE-A-3711991, DE-A-2210667, EP-A-288758 und DE-A-2210687. Diese Verbindungen unterscheiden sich jedoch strukturell von den erfindungsgemäßen Verbindungen stärker als die Verbindungen aus DE-A-4011105. Die überraschend vorteilhafte negativ inotrope Wirkung der speziellen erfindungsgemäßen Verbindungen konnte aus diesem Stand der Technik nicht abgeleitet werden.

Die vorliegende Erfindung betrifft Aryl-Chinolyl-substituierte 1,4-Dihydropyridin-dicarbonsäurederivate der allgemeinen Formel (I)

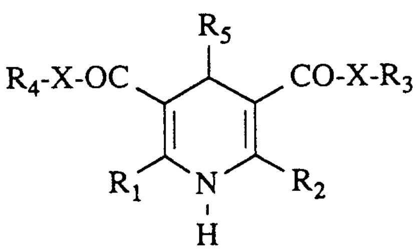

(I)

in welcher

$R^1$ und $R^2$ gleich oder verschieden sind und für Wasserstoff oder für geradkettiges oder verzweigtes Alkyl mit bis zu 6 Kohlenstoffatomen stehen,

oder

einer der beiden Substituenten $R^1$ oder $R^2$ für die $-NH_2$-Gruppe steht,

X für ein Sauerstoffatom oder für die -NH-Gruppe steht,

$R^3$ und $R^4$ gleich oder verschieden sind und für Wasserstoff oder für Cycloalkyl mit 3 bis 6 Kohlenstoffatomen stehen,
oder
für geradkettiges oder verzweigtes Alkenyl oder Alkyl mit jeweils bis zu 10 Kohlenstoffatomen stehen, die gegebenenfalls durch ein Sauerstoff- oder Schwefelatom in der Kette unterbrochen sind und/oder gegebenenfalls durch Carboxy, Halogen, Cyano, Hydroxy, Phenyl, Phenoxy, Benzyloxy oder durch geradkettiges oder verzweigtes Alkoxy, Acyl oder Alkoxycarbonyl mit jeweils bis zu 8 Kohlenstoffatomen oder durch eine Gruppe der Formel $-NR^6R^7$ substituiert sind,
worin

$R^6$ und $R^7$ gleich oder verschieden sind und Wasserstoff, geradkettiges oder verzweigtes Alkyl mit bis zu 6 Kohlenstoffatomen, Benzyl oder Phenyl bedeuten,

$R^5$ für einen Rest der Formel

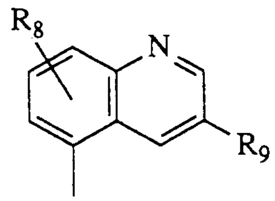

oder

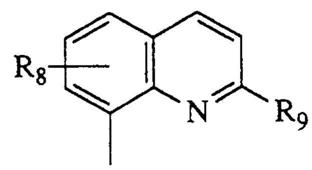

steht,
worin

$R^8$ Wasserstoff, Halogen oder geradkettiges oder verzweigtes Alkyl oder Alkoxy mit jeweils bis zu 6 Kohlenstoffatomen bedeutet,

$R^9$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy oder durch eine Gruppe der Formel $-NR^6R^7$ substituiert ist,
worin

$R^6$ und $R^7$ die oben angegebene Bedeutung haben
oder
2- oder 3-Thienyl bedeutet, das gegebenenfalls durch Halogen substituiert ist

und deren physiologisch unbedenklichen Salze.

Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$ und $R^2$ gleich oder verschieden sind und
für Wasserstoff oder für geradkettiges oder verzweigtes Alkyl mit bis zu 4 Kohlenstoffatomen stehen,

oder
einer der beiden Substituenten $R^1$ oder $R^2$ für die $NH_2$-Gruppe steht,

X für ein Sauerstoffatom oder für die -NH-Gruppe steht,

$R^3$ und $R^4$ gleich oder verschieden sind und
für Wasserstoff, Cyclopropyl, Cyclopentyl oder Cyclohexyl stehen, oder für geradkettiges oder verzweigtes Alkenyl oder Alkyl mit jeweils bis zu 8 Kohlenstoffatomen stehen, die gegebenenfalls durch ein Sauerstoff- oder Schwefelatom in der Kette unterbrochen sind und/oder gegebenenfalls durch Carboxy, Halogen, Cyano, Hydroxy, Phenyl oder durch geradkettiges oder verzweigtes Alkoxy, Acyl oder Alkoxycarbonyl mit jeweils bis zu 6 Kohlenstoffatomen oder durch eine Gruppe der Formel $-NR^6R^7$ substituiert sind,
worin

$R^6$ und $R^7$ gleich oder verschieden sind und Wasserstoff, geradkettiges oder verzweigtes Alkyl mit bis zu 4 Kohlenstoffatomen oder Benzyl bedeuten,

$R^5$ für einen Rest der Formel

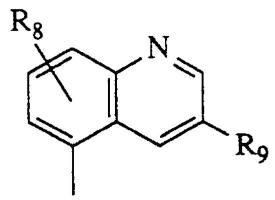

oder

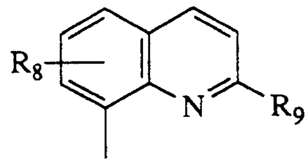

steht,
worin

$R^8$ Wasserstoff, Halogen oder geradkettiges oder verzweigtes Alkyl oder Alkoxy mit jeweils bis zu 4 Kohlenstoffatomen bedeutet,

$R^9$ Phenyl bedeutet, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Benzyl oder durch eine Gruppe der Formel $-NR^6R^7$ substituiert ist,
worin

$R^6$ und $R^7$ die oben angegebene Bedeutung haben
oder
2- oder 3-Thienyl bedeutet, das gegebenenfalls durch Halogen substituiert ist,

und deren physiologisch unbedenklichen Salze.

Besonders bevorzugt sind Verbindungen der allgemeinen Formel (I),
in welcher

$R^1$ und $R^2$ gleich oder verschieden sind und für Wasserstoff, Methyl, Ethyl, Propyl oder Isopropyl stehen, oder einer der beiden Substituenten $R^1$ oder $R^2$ für die $NH_2$-Gruppe steht,

X für ein Sauerstoffatom oder für die -NH-Gruppe steht,

$R^3$ und $R^4$ gleich oder verschieden sind und für Wasserstoff, Cyclopropyl, Cyclopentyl oder für geradkettiges oder verzweigtes Alkyl mit bis zu 6 Kohlenstoffatomen stehen, das gegebenenfalls durch Carboxy, Fluor, Chlor, Cyano, Hydroxy oder durch geradkettiges oder verzweigtes Alkoxy, Acyl oder Alkoxycarbonyl mit jeweils bis zu 4 Kohlenstoffatomen oder durch eine Gruppe der Formel $-NR^6R^7$ substituiert ist,
worin

$R^6$ und $R^7$ gleich oder verschieden sind und Wasserstoff, Methyl, Ethyl oder Benzyl bedeuten,

$R^5$ für eine Gruppe der Formel

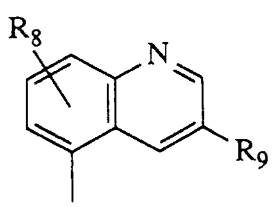

oder

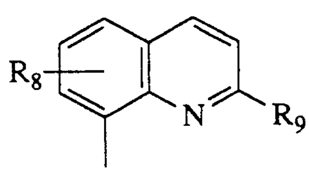

steht,
worin

$R^8$ Wasserstoff, Ethyl oder Methyl bedeutet,

$R^9$ Phenyl bedeutet, das gegebenenfalls bis zu 2-fach gleich oder verschieden durch Fluor, Chlor, Nitro, Trifluormethyl oder durch geradkettiges oder verzweigtes Alkyl oder Alkoxy mit jeweils bis zu 4 Kohlenstoffatomen oder durch eine Gruppe der Formel $-NR^6R^7$ substituiert ist,
worin

$R^6$ und $R^7$ die oben angegebene Bedeutung haben
oder
2- oder 3-Thienyl bedeutet

und deren physiologisch unbedenklichen Salze.

Die Erfindung betrifft auch Verfahren zur Herstellung der erfindungsgemäßen Verbindungen der allgemeinen Formel (I), dadurch gekennzeichnet, daß man

[A] Aldehyde der allgemeinen Formel (II)

$R^5$-CHO (II)

in welcher

$R^5$ die oben angegebene Bedeutung hat,

zunächst mit Acetessigsäurederivaten der allgemeinen Formel (III)

$R^{2'}$-CO-$CH_2$-CO-X-$R^3$ (III)

in welcher

$R^3$ und X die oben angegebene Bedeutung haben, und

$R^{2'}$ die oben angegebene Bedeutung von $R^2$ hat, aber nicht für Amino steht,

gegebenenfalls unter Isolierung der entsprechenden Ylidenverbindung der allgemeinen Formel (IV)

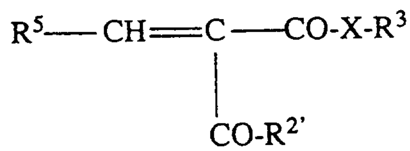

(IV)

in welcher

$R^{2'}$, $R^3$, $R^5$ und X die oben angegebene Bedeutung haben,

umsetzt und anschließend entweder mit Verbindungen der allgemeinen Formel (V)

$R^{1'}$-CO-$CH_2$-CO-X-$R^4$ (V)

in welcher

$R^4$ und X die oben angegebene Bedeutung haben, und

$R^{1'}$ die oben angegebene Bedeutung von $R^1$ hat, aber nicht für Amino steht,

in Anwesenheit von Ammoniak oder Ammoniumsalzen, oder direkt mit den hieraus entstehenden Enaminen der allgemeinen Formel (VI)

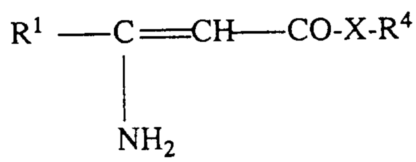

(VI)

in welcher

$R^1$, $R^4$ und X die oben angegebene Bedeutung haben,

gegebenenfalls in Gegenwart von inerten organischen Lösemitteln umsetzt,

oder

[B] die Aldehyde der allgemeinen Formel (II) zunächst mit Verbindungen der allgemeinen Formel (V), gegebenenfalls unter Isolierung der Ylidenverbindungen der allgemeinen Formel (VII)

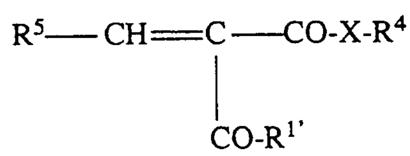

(VII)

in welcher
$R^{1'}$, $R^4$, $R^5$ und X die oben angegebene Bedeutung haben,
umsetzt, und in einem nächsten Schritt mit Verbindungen der allgemeinen Formel (III) in inerten Losemitteln, in Anwesenheit von Ammoniak oder Ammoniumsalzen oder direkt mit den Enaminocarbonsäurederivaten der allgemeinen Formel (VIII)

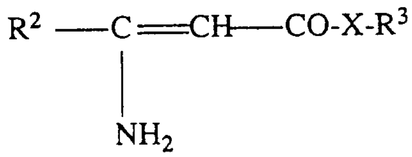

(VIII)

in welcher
$R^2$, $R^3$ und X die oben angegebene Bedeutung haben,
umsetzt,
oder
[C] im Fall, daß $R^4$ nicht Wasserstoff bedeutet, Verbindungen der allgemeinen Formel (I), in welcher $R^1$, $R^2$ und $R^5$ die oben angegebene Bedeutung haben und $R^3$ für Wasserstoff steht, gegebenenfalls über ein reaktives Säurederivat, mit den entsprechenden Alkoholen oder Aminen umsetzt, wobei durch Einsatz der enantiomerenreinen Derivate ($R^3$ = H) die entsprechenden Enantiomere der Verbindungen der Formel (I) erhalten werden.

Die erfindungsgemäßen Verfahren können durch folgendes Formelschema beispielhaft erläutert werden:

[A]

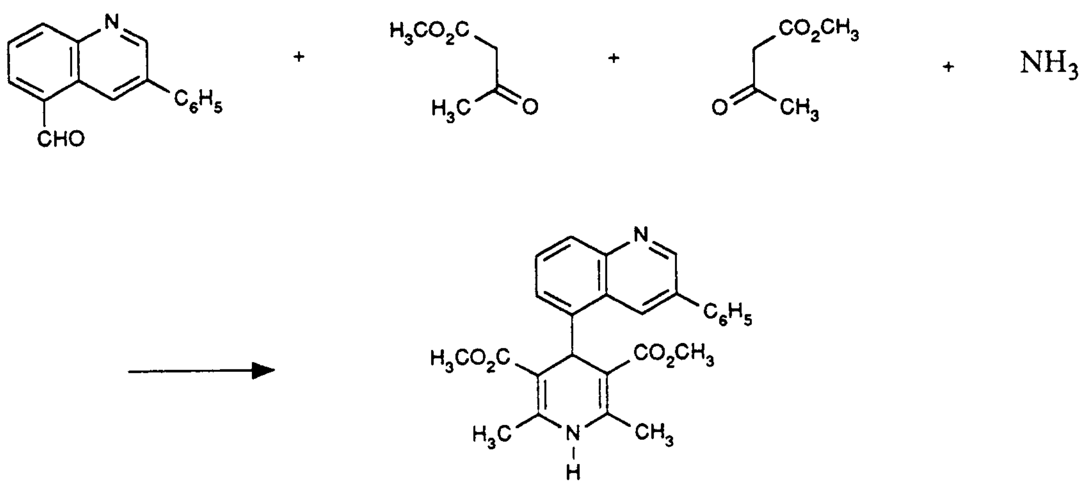

[B]

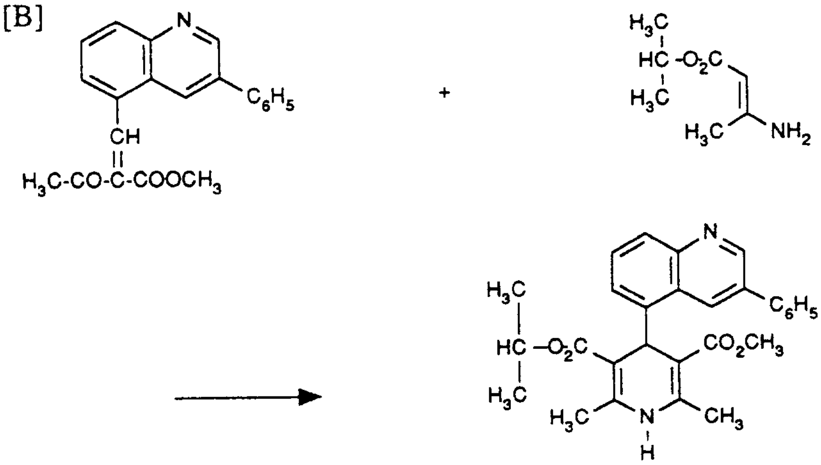

[C]

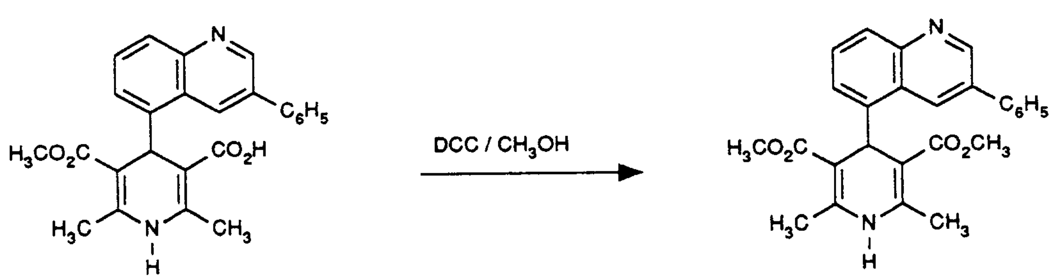


Als Lösemittel für die Verfahren [A] und [B] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante

[A] oder [B] Methanol, Isopropanol, Ethanol und n-Propanol, Acetonitril oder Tetrahydrofuran.

Als Lösemittel für das Verfahren [C] eignen sich die oben aufgeführten Lösemittel mit Ausnahme der Alkohole.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher R4 für einen optischen Esterrest steht, nach üblicher Methode trennt, anschließend entweder direkt umestert oder zuerst die chiralen Carbonsäuren herstellt und dann gegebenenfalls durch Veresterung oder Amidierung die enantiomerenreinen Dihydropyridine herstellt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind teilweise bekannt und können nach üblichen Methoden hergestellt werden, indem man z.B. die entsprechenden Alkyl- oder Hydroxyalkyl-chinoline oxidiert oder die entsprechenden Carboxy-chinoline reduziert (vgl. auch DOS 4 011 105).

Die Acetessigsäure-Derivate der Formel (III) sind bekannt oder können nach üblichen Methoden hergestellt werden [vgl. D. Borrmann, "Umsetzung von Diketonen mit Alkoholen, Phenolen und Mercaptanen", in Houben-Weyl, Methoden der organischen Chemie, Vol. VIII/4, 230 ff. (1968) sowie US 4 948 899].

Die Ylidenverbindungen (IV) und (VII) sind größenteils neu, können aber nach üblichen Methoden hergestellt werden [vgl. H. Dornow und W. Sassenberg, Liebigs Ann. Chem. 602, 14 (1957)].

Die Verbindungen der allgemeinen Formel (V) sind ebenfalls bekannt [vgl. N. Levy, C.W. Scaife, J. Chem. Soc. (London) 1946, 1100; C.D. Hurd, M.E. Nilson, J. Org. Chem. 20, 927 (1955)].

Die Aminocrotonsäurederivate der Formel (VI) und (VIII) sind bekannt oder können nach bekannten Methoden hergestellt werden [S.A. Glickman, A.C. Cope, J. Am. Chem. Soc. 67, 1017 (1946); US 4 948 899].

Die vorstehende Herstellungsverfahren sind lediglich zur Verdeutlichung angegeben.

Die erfindungsgemäßen Verbindungen zeigen ein nicht vorhersehbares, wertvolles pharmakologisches Wirkspektrum. Sie beeinflussen die Kontraktionskraft des Herzens, den Tonus der glatten Muskulatur sowie den Elektrolyt- und Flüssigkeitshaushalt.

Sie können deshalb für die Herstellung von Arzneimitteln zur Behandlung des pathologisch veränderten Blutdrucks und der Herzinsuffizienz, sowie von Coronartherapeutika eingesetzt werden.

Darüber hinaus können sie bei der Herstellung von Arzneimitteln zur Behandlung von Erkrankungen des Kreislaufs und des Flüssigkeitshaushaltes, insbesondere zur Behandlung von Herzrhythmusstörungen, Niereninsuffizienz, Leberzirrhose, Aszites, Lungenödem, Hirnödem, Schwangerschaftsödem, Glaukom oder Diabetes mellitus eingesetzt werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Losung partikelfrei filtriert. Die Losung wird mit Carbogen

($95\%\ O_2$, $5\%\ CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe bei einer Wirkstoffkonzentration von $10^{-6}$ g/ml

| Beispiel Nr. | % Änderung der Ventrikeldruckamplitude |
|---|---|
| 3 | -100% |
| 4 | -66% |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindung

Beispiel I

3-(3-Phenyl-chinolin-5-yliden)-2-oxo-buttersäure-2-(N-benzyl-N-methylamino)-ethylester

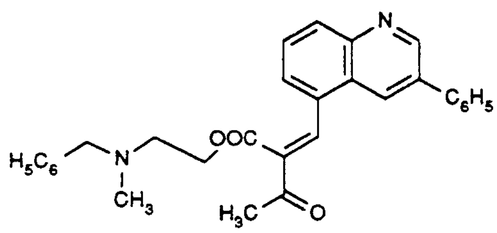

11,65 g (50 mmol) 3-Phenyl-chinolin-5-aldehyd werden in 250 ml Methylenchlorid mit 12,5 g (50 mmol) 2-Oxo-buttersäure-2-(N-benzyl-N-methylamino)-ethylester und einer katalytischen Menge Piperidinacetat über Nacht am Wasserabscheider gekocht. Es wird abgekühlt, zweimal mit Wasser ausgeschüttelt, getrocknet und eingeengt. Das erhaltene Rohprodukt wird durch Flash-Chromatographie mit Toluol/Essigester-Gemischen gereinigt. Man erhält 15,6 g (67,2% der Theorie) eines leicht gefärbten Öls.

Herstellungsbeispiele

Beispiel 1

1,4-Dihydro-2,6-dimethyl-4-(3-phenyl-chinolin-5-yl)-pyridin-3,5-dicarbonsäuredimethylester

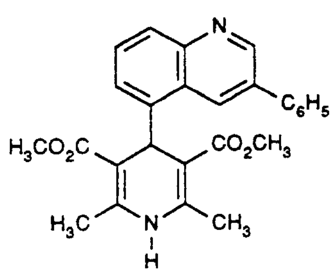

2,33 g (10 mmol) 3-Phenyl-chinolin-5-aldehyd werden in 20 ml Methanol mit 2,3 g Acetessigsäuremethylester und 1,5 ml 25% Ammoniak-Lösung 16 Stunden gekocht. Es werden weitere 0,75 ml Ammoniak zugegeben, 24 Stunden gekocht, abgekühlt; die ausgefallene Substanz wird abgesaugt und mit wenig Methanol gewaschen. Man erhält 2,91 g farblose Kristalle vom Schmelzpunkt 265 °C.

Beispiel 2

1,4-Dihydro-2,6-dimethyl-4-(3-phenyl-chinolin-5-yl)-pyridin-3,5-dicarbonsäure-3-[2-(N-benzyl-N-methylamino)-ethylester-5-methylester

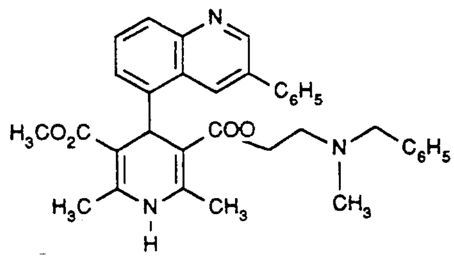

4,64 g (10 mmol) der Verbindung aus Beispiel I werden in 20 ml Isopropanol mit 1,15 g (10 mmol) 3-Aminocrotonsäuremethylester 18 Stunden zum Rückfluß erhitzt.

Es wird abgekühlt, eingeengt und durch Flash-Chromatographie mit Methylenchlorid/Essigester-Gemischen gereinigt. Die sauberen Fraktionen werden vereint, eingeengt und durch Verrühren mit Acetonitril kristallisiert. Es wird abgesaugt und mit Acetonitril gewaschen. Man erhält 3,3 g einer farblosen Substanz vom Schmelzpunkt 169-171 °C.

In Analogie zu den Vorschriften der Beispiele 1 und 2 werden die in den Tabellen 1-5 aufgeführten Beispiele hergestellt:

Tabelle 1:

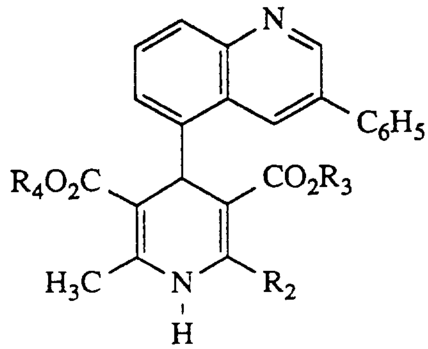

| Bsp.-Nr. | $R^2$ | $R^3$ | $R^4$ | F°C |
|---|---|---|---|---|
| 3 | $-CH_3$ | $-C_2H_5$ | $-C_2H_5$ | 268 |
| 4 | $-CH_3$ | $-(CH_2)_2CN$ | $-CH_3$ | 199 |
| 5 | $-CH_3$ | -H | $-CH_3$ | 197 |
| 6 | $-CH_3$ | $-(CH_2)_2CN$ | $-CH(CH_3)_2$ | 169 |
| 7 | $-CH_3$ | $-(CH_2)_2CN$ | $-C_2H_5$ | 196 |
| 8 | $-CH_3$ | -H | $-CH(CH_3)_2$ | 146 |
| 9 | $-CH_3$ | -H | $-C_2H_5$ | 149 |
| 10 | $-C_2H_5$ | $-C_2H_5$ | $-CH_3$ | 220 |
| 11 | H | $-C_2H_5$ | $-CH_3$ | 253 |
| 12 | $-CH_3$ | $-CH(CH_3)_2$ | $-CH_3$ | 205 |
| 13 | $-CH_3$ | $-(CH_2)_2OCH_3$ | $-CH(CH_3)_2$ | 185 |
| 14 | $-NH_2$ | $-CH_3$ | $-CH(CH_3)_2$ | 233 |

Tabelle 2:

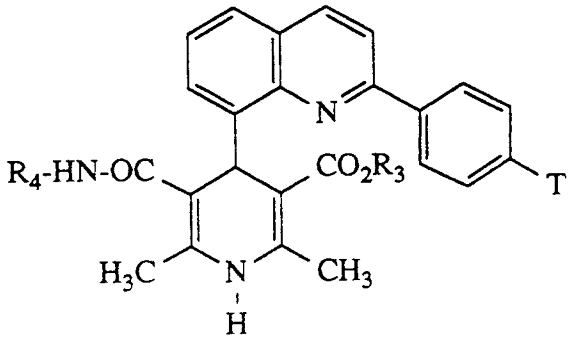

| Bsp.-Nr. | $R^3$ | $R^4$ | T | F°C |
|---|---|---|---|---|
| 15 | 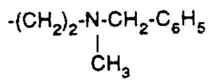 | 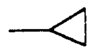 | F | 209 |
| 16 | $-CH_3$ | 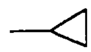 | F | 277 |
| 17 | 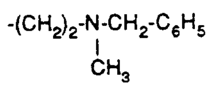 | $-C_2H_5$ | F | 184 |
| 18 | $-CH_3$ | $-C_2H_5$ | F | 249 |
| 19 | 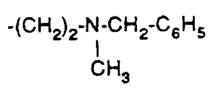 | $-C_2H_5$ | H | 176 |
| 20 | 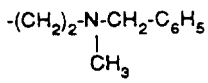 | 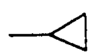 | H | 193 |
| 21 | $-CH_3$ | $-C_2H_5$ | H | 237-238 |
| 22 | $-CH_3$ | 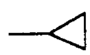 | H | 248-250 |

Tabelle 3:

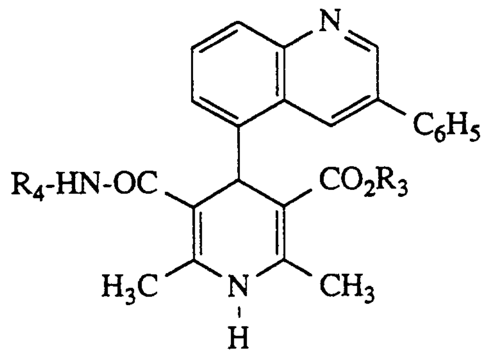

| Bsp.-Nr. | $R^3$ | $R^4$ | F°C |
|---|---|---|---|
| 23 | $-CH_3$ | 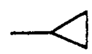 | 239 |

Tabelle 4:

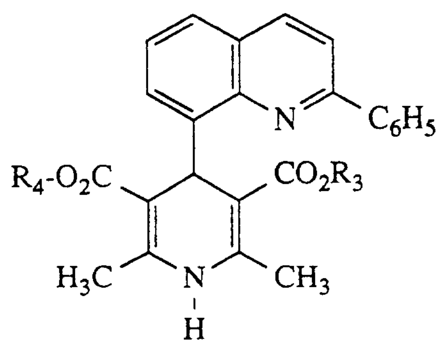

| Bsp.-Nr. | $R^3$ | $R^4$ | F°C |
|---|---|---|---|
| 24 | $-CH_3$ | $-CH_3$ | 216-217 |

Tabelle 5:

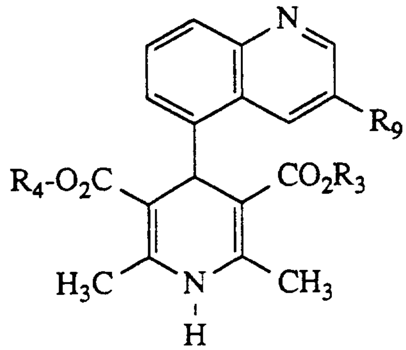

| Bsp.-Nr. | $R^3$ | $R^4$ | $R^9$ | F°C |
|---|---|---|---|---|
| 25 | $-CH_3$ | $-CH(CH_3)_2$ | 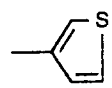 | 195 |
| 26 | $-CH(CH_3)_2$ | $-CH(CH_3)_2$ | 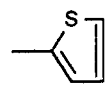 | 204 |
| 27 | $-CH_3$ | $-CH_3$ | 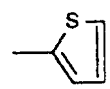 | 269 |

Tabelle 6:

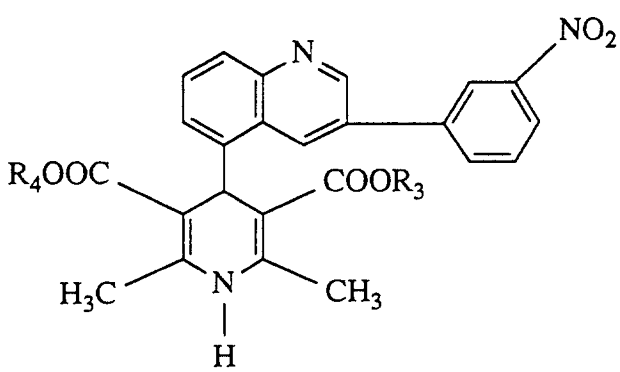

| Bsp.-Nr. | $R^3$ | $R^4$ | F°C |
|---|---|---|---|
| 28 | $-CH_3$ | $-CH_3$ | 228 |

## Patentansprüche

1. Aryl-Chinolyl-substituierte 1,4-Dihydropyridin-dicarbonsäurederivate der allgemeinen Formel (I)

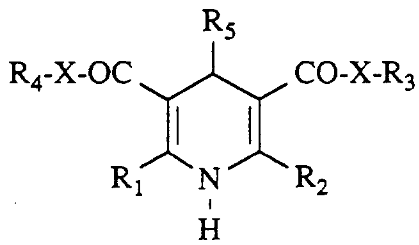

(I)

in welcher

$R^1$ und $R^2$ gleich oder verschieden sind und für Wasserstoff oder für geradkettiges oder verzweigtes Alkyl mit bis zu 6 Kohlenstoffatomen stehen,

oder

einer der beiden Substituenten $R^1$ oder $R^2$ für die $-NH_2$-Gruppe steht,

X für ein Sauerstoffatom oder für die -NH-Gruppe steht,

$R^3$ und $R^4$ gleich oder verschieden sind und für Wasserstoff oder für Cycloalkyl mit 3 bis 6 Kohlenstoffatomen stehen, oder für geradkettiges oder verzweigtes Alkenyl oder Alkyl mit jeweils bis zu 10 Kohlenstoffatomen stehen, die gegebenenfalls durch ein Sauerstoff- oder Schwefelatom in der Kette unterbrochen sind und/oder gegebenenfalls durch Carboxy, Halogen, Cyano, Hydroxy, Phenyl, Phenoxy, Benzyloxy oder durch geradkettiges oder verzweigtes Alkoxy, Acyl oder Alkoxycarbonyl mit jeweils bis zu 8 Kohlenstoffatomen oder durch eine Gruppe der Formel $-NR^6R^7$ substituiert sind,

worin

$R^6$ und $R^7$ gleich oder verschieden sind und Wasserstoff, geradkettiges oder verzweigtes Alkyl mit bis zu 6 Kohlenstoffatomen, Benzyl oder Phenyl bedeuten,

$R^5$ für einen Rest der Formel

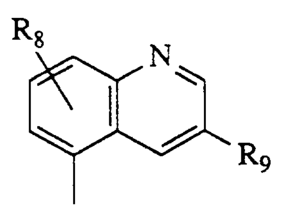

oder

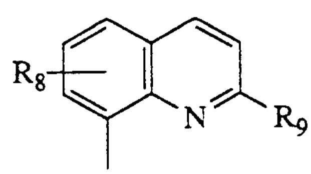

steht,

worin

$R^8$ Wasserstoff, Halogen oder geradkettiges oder verzweigtes Alkyl oder Alkoxy mit jeweils bis zu 6 Kohlenstoffatomen bedeutet,

$R^9$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann, oder durch geradkettiges oder verzweigtes Alkoxy oder Alkoxycarbonyl mit jeweils bis zu 8 Kohlenstoffatomen, Carboxy oder durch eine Gruppe der Formel $-NR^6R^7$ substituiert ist,

worin

$R^6$ und $R^7$ die oben angegebene Bedeutung haben

oder

2- oder 3-Thienyl bedeutet, das gegebenenfalls durch Halogen substituiert ist
und deren physiologisch unbedenklichen Salze.

**2.** Verbindungen der allgemeinen Formel (I) gemäß Anspruch 1, in welcher

$R^1$ und $R^2$ gleich oder verschieden sind und
für Wasserstoff oder für geradkettiges oder verzweigtes Alkyl mit bis zu 4 Kohlenstoffatomen stehen,

oder

einer der beiden Substituenten $R^1$ oder $R^2$ für die $NH_2$-Gruppe steht,

X für ein Sauerstoffatom oder für die -NH-Gruppe steht,

$R^3$ und $R^4$ gleich oder verschieden sind und
für Wasserstoff, Cyclopropyl, Cyclopentyl oder Cyclohexyl stehen, oder
für geradkettiges oder verzweigtes Alkenyl oder Alkyl mit jeweils bis zu 8 Kohlenstoffatomen stehen, die gegebenenfalls durch ein Sauerstoff- oder Schwefelatom in der Kette unterbrochen sind und/oder gegebenenfalls durch Carboxy, Halogen, Cyano, Hydroxy, Phenyl oder durch geradkettiges oder verzweigtes Alkoxy, Acyl oder Alkoxycarbonyl mit jeweils bis zu 6 Kohlenstoffatomen oder durch eine Gruppe der Formel $-NR^6R^7$ substituiert sind,
worin

$R^6$ und $R^7$ gleich oder verschieden sind und Wasserstoff, geradkettiges oder verzweigtes Alkyl mit bis zu 4 Kohlenstoffatomen oder Benzyl bedeuten,

$R^5$ für einen Rest der Formel

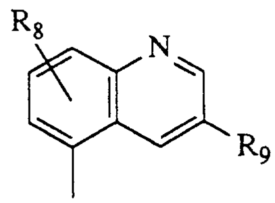

oder

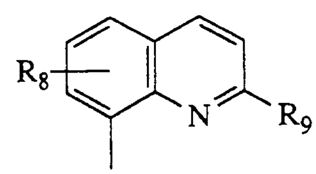

steht,
worin

$R^8$ Wasserstoff, Halogen oder geradkettiges oder verzweigtes Alkyl oder Alkoxy mit jeweils bis zu 4 Kohlenstoffatomen bedeutet,

$R^9$ Phenyl bedeutet, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Benzyl oder durch eine Gruppe der Formel $-NR^6R^7$ substituiert ist,
worin

$R^6$ und $R^7$ die oben angegebene Bedeutung haben
oder
2- oder 3-Thienyl bedeutet, das gegebenenfalls durch Halogen substituiert ist,

und deren physiologisch unbedenklichen Salze.

**3.** Verbindungen der allgemeinen Formel (I) gemäß Anspruch 1, in welcher

$R^1$ und $R^2$ gleich oder verschieden sind und
für Wasserstoff, Methyl, Ethyl, Propyl oder Isopropyl stehen, oder einer der beiden Substituenten $R^1$ oder $R^2$ für die $NH_2$-Gruppe steht,

X für ein Sauerstoffatom oder für die -NH-Gruppe steht,

$R^3$ und $R^4$ gleich oder verschieden sind und
für Wasserstoff, Cyclopropyl, Cyclopentyl oder für geradkettiges oder verzweigtes Alkyl mit bis zu 6 Kohlenstoffatomen stehen, das gegebenenfalls durch Carboxy, Fluor, Chlor, Cyano, Hydroxy oder durch geradkettiges oder verzweigtes Alkoxy, Acyl oder Alkoxycarbonyl mit jeweils bis zu 4 Kohlenstoffatomen oder durch eine Gruppe der Formel $-NR^6R^7$ substituiert ist,
worin

$R^6$ und $R^7$ gleich oder verschieden sind und Wasserstoff, Methyl, Ethyl oder Benzyl bedeuten,
$R^5$ für eine Gruppe der Formel

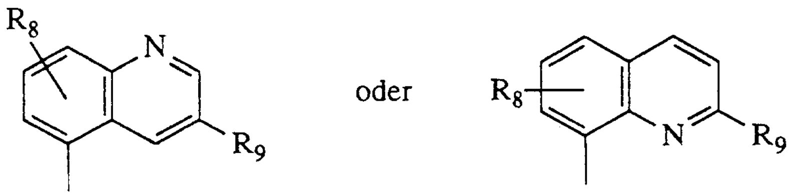

steht,
worin
$R^8$ Wasserstoff, Ethyl oder Methyl bedeutet,
$R^9$ Phenyl bedeutet, das gegebenenfalls bis zu 2-fach gleich oder verschieden durch Fluor, Chlor, Nitro, Trifluormethyl oder durch geradkettiges oder verzweigtes Alkyl oder Alkoxy mit jeweils bis zu 4 Kohlenstoffatomen oder durch eine Gruppe der Formel $-NR^6R^7$ substituiert ist,
worin
$R^6$ und $R^7$ die oben angegebene Bedeutung haben
oder
2- oder 3-Thienyl bedeutet,
und deren physiologisch unbedenklichen Salze.

**4.** Verbindungen der allgemeinen Formel (I) gemäß Anspruch 1 zur Verwendung bei der Bekämpfung von Kreislauferkrankungen.

**5.** Verfahren zur Herstellung von Verbindungen der allgemeinen Formel (I)

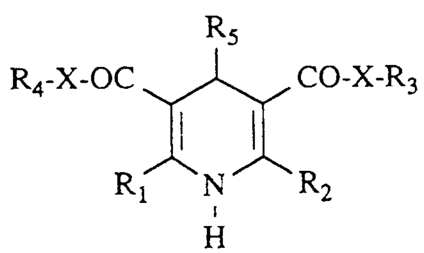

(I)

in welcher
$R^1$ und $R^2$ gleich oder verschieden sind und für Wasserstoff oder für geradkettiges oder verzweigtes Alkyl mit bis zu 6 Kohlenstoffatomen stehen,
oder
einer der beiden Substituenten $R^1$ oder $R^2$ für die $-NH_2$-Gruppe steht,
X für ein Sauerstoffatom oder für die -NH-Gruppe steht,
$R^3$ und $R^4$ gleich oder verschieden sind und für Wasserstoff oder für Cycloalkyl mit 3 bis 6 Kohlenstoffatomen stehen, oder für geradkettiges oder verzweigtes Alkenyl oder Alkyl mit jeweils bis zu 10 Kohlenstoffatomen stehen, die gegebenenfalls durch ein Sauerstoff- oder Schwefelatom in der Kette unterbrochen sind und/oder gegebenenfalls durch Carboxy, Halogen, Cyano, Hydroxy, Phenyl, Phenoxy, Benzyloxy oder durch geradkettiges oder verzweigtes Alkoxy, Acyl oder Alkoxycarbonyl mit jeweils bis zu 8 Kohlenstoffatomen oder durch eine Gruppe der Formel $-NR^6R^7$ substituiert sind,
worin
$R^6$ und $R^7$ gleich oder verschieden sind und Wasserstoff, geradkettiges oder verzweigtes Alkyl mit bis zu 6 Kohlenstoffatomen, Benzyl oder Phenyl bedeuten,

$R^5$ für einen Rest der Formel

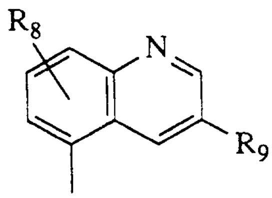

oder

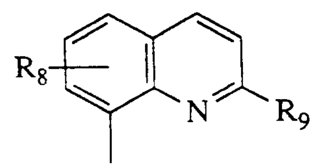

steht,
worin

$R^8$ Wasserstoff, Halogen oder geradkettiges oder verzweigtes Alkyl oder Alkoxy mit jeweils bis zu 6 Kohlenstoffatomen bedeutet,

$R^9$ Aryl mit 6 bis 10 Kohlenstoffatomen bedeutet, das gegebenenfalls bis zu 3-fach gleich oder verschieden durch Halogen, Nitro, Cyano, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann, oder durch geradkettiges oder verzweigtes Alkoxy oder Alkoxycarbonyl mit jeweils bis zu 8 Kohlenstoffatomen, Carboxy oder durch eine Gruppe der Formel $-NR^6R^7$ substituiert ist,
worin

$R^6$ und $R^7$ die oben angegebene Bedeutung haben
oder
2- oder 3-Thienyl bedeutet, das gegebenenfalls durch Halogen substituiert ist

und deren physiologisch unbedenklichen Salze,
dadurch gekennzeichnet, daß man

[A] Aldehyde der allgemeinen Formel (II)

$R^5$-CHO (II)

in welcher
$R^5$ die oben angegebene Bedeutung hat,
zunächst mit Acetessigsäurederivaten der allgemeinen Formel (III)

$R^{2'}$-CO-$CH_2$-CO-X-$R^3$ (III)

in welcher
$R^3$ und X die oben angegebene Bedeutung haben, und
$R^{2'}$ die oben angegebene Bedeutung von $R^2$ hat, aber nicht für Amino steht,
gegebenenfalls unter Isolierung der entsprechenden Ylidenverbindung der allgemeinen Formel (IV)

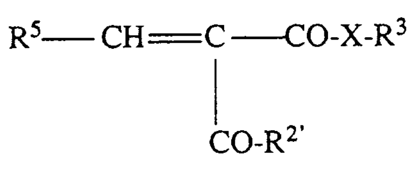

(IV)

in welcher
$R^{2'}$, $R^3$, $R^5$ und X die oben angegebene Bedeutung haben,
umsetzt und anschließend entweder mit Verbindungen der allgemeinen Formel (V)

$R^{1'}$-CO-$CH_2$-CO-X-$R^4$ (V)

in welcher
$R^4$ und X die oben angegebene Bedeutung haben, und

$R^{1'}$ die oben angegebene Bedeutung von $R^1$ hat, aber nicht für Amino steht, in Anwesenheit von Ammoniak oder Ammoniumsalzen, oder direkt mit den Enaminen der allgemeinen Formel (VI)

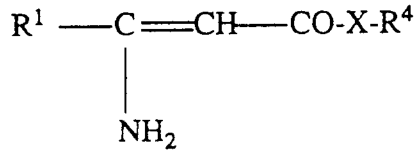

(VI)

in welcher
$R^1$, $R^4$ und X die oben angegebene Bedeutung haben,
gegebenenfalls in Gegenwart von inerten organischen Lösemitteln umsetzt,
oder
[B] die Aldehyde der allgemeinen Formel (II) zunächst mit Verbindungen der allgemeinen Formel (V), gegebenenfalls unter Isolierung der Ylidenverbindungen der allgemeinen Formel (VII)

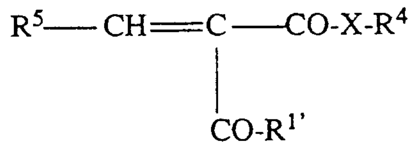

(VII)

in welcher
$R^{1'}$, $R^4$, $R^5$ und X die oben angegebene Bedeutung haben,
umsetzt, und in einem nächsten Schritt mit Verbindungen der allgemeinen Formel (III) in inerten Losemitteln, in Anwesenheit von Ammoniak oder Ammoniumsalzen oder direkt mit den Enaminocarbonsäurederivaten der allgemeinen Formel (VIII)

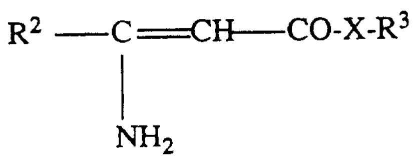

(VIII)

in welcher
$R^2$, $R^3$ und X die oben angegebene Bedeutung haben,
umsetzt,
oder
[C] im Fall, daß $R^4$ nicht Wasserstoff bedeutet, Verbindungen der allgemeinen Formel (I), in welcher $R^1$, $R^2$ und $R^5$ die oben angegebene Bedeutung haben und $R^3$ für Wasserstoff steht, gegebenenfalls über ein reaktives Säurederivat, mit den entsprechenden Alkoholen oder Aminen umsetzt, wobei durch Einsatz der enantiomerenreinen Derivate ($R^3$ = H) die entsprechenden Enantiomere der Verbindungen der Formel (I) erhalten werden.

**6.** Arzneimittel enthaltend mindestens eine Verbindung der allgemeinen Formel (I) gemäß Anspruch 1.

**7.** Verfahren zur Herstellung von Arzneimitteln, dadurch gekennzeichnet, daß man Verbindungen der allgemeinen Formel (I) gemäß Anspruch 1 gegebenenfalls mit üblichen Hilfs- und Trägerstoffen in eine geeignete Applikationsform überführt.

**8.** Verwendung von Verbindungen der allgemeinen Formel (I) gemäß Anspruch 1 bei der Herstellung von Arzneimitteln zur Behandlung von Erkrankungen des Kreislaufs und des Flüssigkeitshaushaltes.

## Claims

**1.** Aryl-quinolyl-substituted 1,4-dihydropyridine-dicarboxylic acid derivatives of general formula (I):

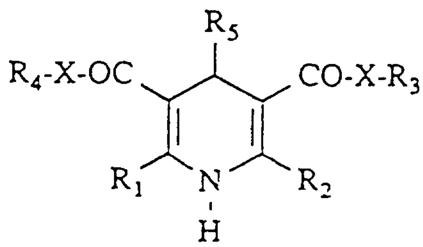

(I)

in which

$R^1$ and $R^2$ are identical or different and are hydrogen or linear or branched alkyl having up to 6 carbon atoms,

or

one of the two substituents $R^1$ or $R^2$ is the $-NH_2$ group,

X is an oxygen atom or the -NH- group,

$R^3$ and $R^4$ are identical or different and are hydrogen or cycloalkyl having 3 to 6 carbon atoms, or are linear or branched alkenyl or alkyl each having up to 10 carbon atoms which may be interrupted by an oxygen or sulphur atom in the chain and/or are unsubstituted or substituted by carboxyl, halogen, cyano, hydroxyl, phenyl, phenoxy or benzyloxy, or by linear or branched alkoxy, acyl or alkoxycarbonyl each having up to 8 carbon atoms, or by a group of the formula $-NR^6R^7$,

wherein

$R^6$ and $R^7$ are identical or different and are hydrogen, linear or branched alkyl having up to 6 carbon atoms, benzyl or phenyl,

$R^5$ is a radical of the formula

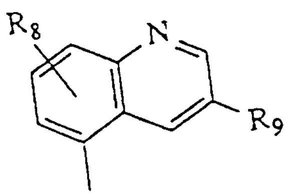

or

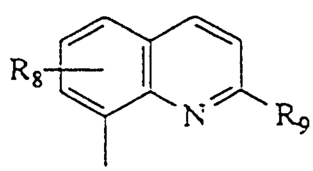

wherein

$R^8$ is hydrogen, halogen, or linear or branched alkyl or alkoxy each having up to 6 carbon atoms,

$R^9$ is aryl having 6 to 10 carbon atoms which is unsubstituted or substituted by up to 3 identical or different substituents selected from the group comprising halogen, nitro, cyano, trifluoromethyl, trifluoromethoxy and trifluoromethylthio, or by linear or branched alkyl having up to 8 carbon atoms, which in turn can be substituted by aryl having 6 to 10 carbon atoms, or by linear or branched alkoxy or alkoxycarbonyl each having up to 8 carbon atoms, by carboxyl or by a group of the formula $-NR^6R^7$,

wherein

$R^6$ and $R^7$ are as defined above,

or

$R^9$ is 2- or 3-thienyl which is unsubstituted or substituted by halogen,

and their physiologically acceptable salts.

**2.** Compounds of general formula (I) according to Claim 1 in which

$R^1$ and $R^2$ are identical or different and are hydrogen or linear or branched alkyl having up to 4 carbon atoms,

or

one of the two substituents $R^1$ or $R^2$ is the $-NH_2$ group,

X is an oxygen atom or the -NH- group,

$R^3$ and $R^4$ are identical or different and are hydrogen, cyclopropyl, cyclopentyl or cyclohexyl, or are linear or branched alkenyl or alkyl each having up to 8 carbon atoms which may be interrupted by an oxygen or sulphur atom in the chain and/or are unsubstituted or substituted by carboxyl, halogen, cyano, hydroxyl or phenyl, or by linear or branched alkoxy, acyl or alkoxycarbonyl each having up to 6 carbon atoms, or by a group of the formula $-NR^6R^7$,

wherein

$R^6$ and $R^7$ are identical or different and are hydrogen, linear or branched alkyl having up to 4 carbon atoms, or benzyl,

$R^5$ is a radical of the formula

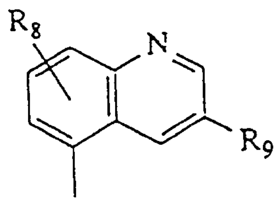

or

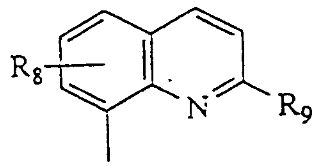

wherein

$R^8$ is hydrogen, halogen or linear or branched alkyl or alkoxy each having up to 4 carbon atoms,

$R^9$ is phenyl which is unsubstituted or substituted by up to 2 identical or different substituents selected from the group comprising halogen, nitro, cyano and trifluoromethyl, or by linear or branched alkyl or alkoxy each having up to 6 carbon atoms, by benzyl or by a group of the formula $-NR^6R^7$,

wherein

$R^6$ and $R^7$ are as defined above,

or

$R^9$ is 2- or 3-thienyl which is unsubstituted or substituted by halogen,

and their physiologically acceptable salts.

**3.** Compounds of general formula (I) according to Claim 1 in which

$R^1$ and $R^2$ are identical or different and are hydrogen, methyl, ethyl, propyl or isopropyl, or one of the two substituents $R^1$ or $R^2$ is the $-NH_2$ group,

X is an oxygen atom or the -NH- group,

$R^3$ and $R^4$ are identical or different and are hydrogen, cyclopropyl, cyclopentyl or linear or branched alkyl having up to 6 carbon atoms, which is unsubstituted or substituted by carboxyl, fluorine, chlorine, cyano or hydroxyl, or by linear or branched alkoxy, acyl or alkoxycarbonyl each having up to 4 carbon atoms, or by a group of the formula $-NR^6R^7$,

wherein

$R^6$ and $R^7$ are identical or different and are hydrogen, methyl, ethyl or benzyl,

$R^5$ is a group of the formula

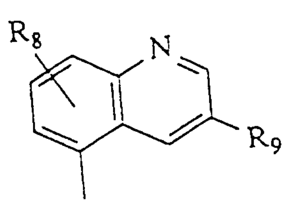

or

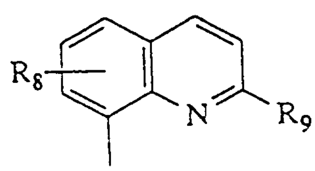

wherein

$R^8$ is hydrogen, ethyl or methyl,
and
$R^9$ is phenyl which is unsubstituted or substituted by up to 2 identical or different substituents selected from the group comprising fluorine, chlorine, nitro and trifluoromethyl, or by linear or branched alkyl or alkoxy each having up to 4 carbon atoms, or by a group of the formula $-NR^6R^7$,
wherein
$R^6$ and $R^7$ are as defined above,
or
$R^9$ is 2- or 3-thienyl,
and their physiologically acceptable salts.

**4.** Compounds of general formula (I) according to Claim 1 for use in the control of circulatory diseases.

**5.** Process for the preparation of compounds of general formula (I)

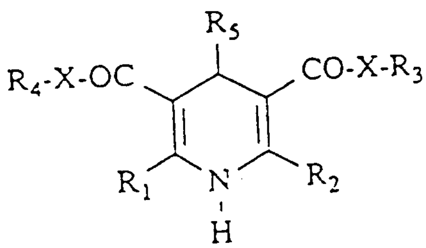

(I)

in which
$R^1$ and $R^2$ are identical or different and are hydrogen or linear or branched alkyl having up to 6 carbon atoms,
or
one of the two substituents $R^1$ or $R^2$ is the $-NH_2$ group,
X is an oxygen atom or the -NH- group,
$R^3$ and $R^4$ are identical or different and are hydrogen or cycloalkyl having 3 to 6 carbon atoms, or are linear or branched alkenyl or alkyl each having up to 10 carbon atoms which may be interrupted by an oxygen or sulphur atom in the chain and/or are unsubstituted or substituted by carboxyl, halogen, cyano, hydroxyl, phenyl, phenoxy or benzyloxy, or by linear or branched alkoxy, acyl or alkoxycarbonyl each having up to 8 carbon atoms, or by a group of the formula $-NR^6R^7$,
wherein
$R^6$ and $R^7$ are identical or different and are hydrogen, linear or branched alkyl having up to 6 carbon atoms, benzyl or phenyl,
$R^5$ is a radical of the formula

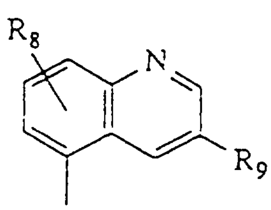

or

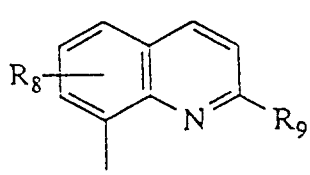

wherein
$R^8$ is hydrogen, halogen or linear or branched alkyl or alkoxy each having up to 6 carbon atoms,
$R^9$ is aryl having 6 to 10 carbon atoms which is unsubstituted or substituted by up to 3 identical or different substituents selected from the group comprising halogen, nitro,

cyano, trifluoromethyl, trifluoromethoxy and trifluoromethylthio, or by linear or branched alkyl having up to 8 carbon atoms, which in turn can be substituted by aryl having 6 to 10 carbon atoms, or by linear or branched alkoxy or alkoxycarbonyl each having up to 8 carbon atoms, by carboxyl or by a group of the formula $-NR^6R^7$, wherein

$R^6$ and $R^7$ are as defined above,

or

$R^9$ is 2- or 3-thienyl which is unsubstituted or substituted by halogen,

and their physiologically acceptable salts,
characterized in that

[A] aldehydes of general formula (II):

$R^5$-CHO (II)

in which
$R^5$ is as defined above,
are first reacted with acetoacetic acid derivatives of general formula (III):

$R^{2'}$-CO-$CH_2$-CO-X-$R^3$ (III)

in which
$R^3$ and X are as defined above, and
$R^{2'}$ is as defined above for $R^2$, but is not amino,
if appropriate with isolation of the corresponding ylidene compound of general formula (IV):

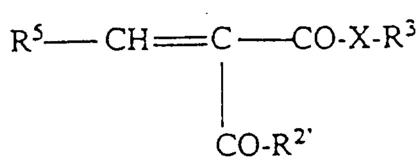

(IV)

in which
$R^{2'}$, $R^3$, $R^5$ and X are as defined above,
and then reacted either with compounds of general formula (V):

$R^{1'}$-CO-$CH_2$-CO-X-$R^4$ (V)

in which
$R^4$ and X are as defined above, and
$R^{1'}$ is as defined above for $R^1$, but is not amino,
in the presence of ammonia or ammonium salts, or directly with the enamines of general formula (VI):

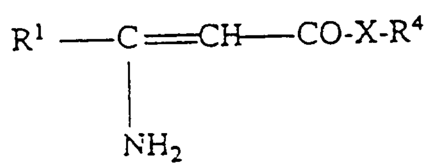

(VI)

in which
$R^1$, $R^4$ and X are as defined above,
if appropriate in the presence of inert organic solvents,
or

[B] the aldehydes of general formula (II) are first reacted with compounds of general formula (V), if appropriate with isolation of the ylidene compounds of general formula (VII):

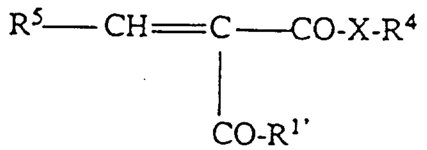

(VII)

in which
$R^{1'}$, $R^4$, $R^5$ and X are as defined above,
and reacted in a subsequent step with compounds of general formula (III) in inert solvents, in the presence of ammonia or ammonium salts, or directly with the enamino carboxylic acid derivatives of general formula (VIII):

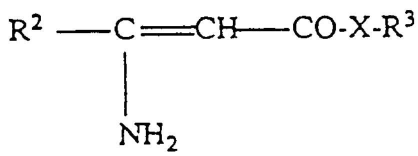

(VIII)

in which
$R^2$, $R^3$ and X are as defined above,
or
[C] in the case where $R^4$ is not hydrogen, compounds of general formula (I) in which $R^1$, $R^2$ and $R^5$ are as defined above and $R^3$ is hydrogen are reacted with the appropriate alcohols or amines, if appropriate via a reactive acid derivative, the use of the enantiomerically pure derivatives ($R^3$ = H) giving the corresponding enantiomers of the compounds of formula (I).

**6.** Drug containing at least one compound of general formula (I) according to Claim 1.

**7.** Process for the preparation of drugs, characterized in that compounds of general formula (I) according to Claim 1 are converted to a suitable form of administration, if appropriate with conventional adjuncts and excipients.

**8.** Use of compounds of general formula (I) according to Claim 1 for the preparation of drugs for the treatment of diseases of the circulation and fluid balance.

**Revendications**

**1.** Dérivés d'acides 1,4-dihydropyridine-dicarboxyliques à substituant aryl-quinolyle de formule générale (I)

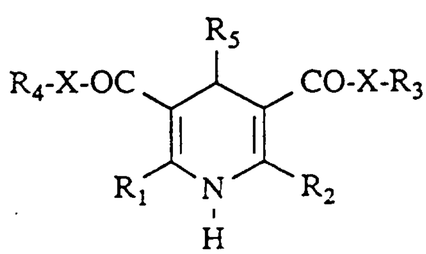

(I)

dans laquelle
$R^1$ et $R^2$ sont égaux ou différents et représentent de l'hydrogène ou un groupe alkyle linéaire ou ramifié ayant jusqu'à 6 atomes de carbone,
ou bien
l'un des deux substituants $R^1$ et $R^2$ est un groupe $-NH_2$,
X est un atome d'oxygène ou le groupe -NH,

$R^3$ et $R^4$ sont égaux ou différents et représentent de l'hydrogène ou un groupe cycloalkyle ayant 3 à 6 atomes de carbone, ou bien un groupe alcényle ou un groupe alkyle linéaire ou ramifié ayant chacun jusqu'à 10 atomes de carbone, dont la chaîne est interrompue le cas échéant par un atome d'oxygène ou de soufre et/ou qui sont substitués le cas échéant par un radical carboxy, halogéno, cyano, hydroxy, phényle, phénoxy, benzyloxy ou par un radical alkoxy, acyle ou alkoxycarbonyle linéaire ou ramifié ayant jusqu'à 8 atomes de carbone ou par un groupe de formule $-NR^6R^7$,
où
$R^6$ et $R^7$ sont égaux ou différents et représentent de l'hydrogène, un groupe alkyle linéaire ou ramifié ayant jusqu'à 6 atomes de carbone, un groupe benzyle ou phényle,
$R^5$ est un reste de formule

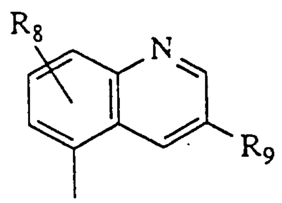

ou

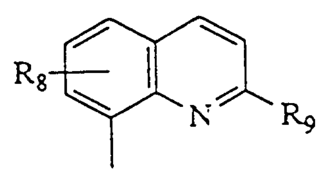

où
$R^8$ représente de l'hydrogène, un halogène ou un groupe alkyle ou alkoxy linéaire ou ramifié ayant jusqu'à 6 atomes de carbone,
$R^9$ est un groupe aryle ayant 6 à 10 atomes de carbone, qui peut être substitué le cas échéant jusqu'à 3 trois fois identiques ou différentes par un radical halogéno, nitro, cyano, trifluorométhyle, trifluorométhoxy, trifluorométhylthio ou par un groupe alkyle linéaire ou ramifié ayant jusqu'à 8 atomes de carbone, qui peut être substitué quant à lui par un radical aryle ayant jusqu'à 10 atomes de carbone, ou bien par un radical alkoxy ou alkoxycarbonyle linéaire ou ramifié ayant chacun jusqu'à 8 atomes de carbone, un radical carboxy ou un groupe de formule $-NR^6R^7$,
où
$R^6$ et $R^7$ ont la définition indiquée ci-dessus,
ou représente un groupe 2- ou 3-thiényle qui est éventuellement substitué par un halogène

et leurs sels acceptables du point de vue physiologique.

**2.** Composés de formule générale (I) suivant la revendication 1, dans laquelle
$R^1$ et $R^2$ sont égaux ou différents et représentent de l'hydrogène ou un groupe alkyle linéaire ou ramifié ayant jusqu'à 4 atomes de carbone,
ou bien
l'un des deux substituants $R^1$ et $R^2$ est un groupe $NH_2$,
X est un atome d'oxygène ou le groupe -NH,
$R^3$ et $R^4$ sont égaux ou différents et représentent de l'hydrogène, un groupe cyclopropyle, cyclopentyle, ou bien
un groupe alcényle ou groupe alkyle linéaire ou ramifié ayant chacun jusqu'à 8 atomes de carbone, dont la chaîne est interrompue le cas échéant par un atome d'oxygène ou de soufre et/ou qui sont éventuellement substitués par un radical carboxy, halogéno, cyano, hydroxy, phényle ou par un radical alkoxy, acyle ou alkoxycarbonyle linéaire ou ramifié ayant jusqu'à 6 atomes de carbone ou par un groupe de formule $-NR^6R^7$,
où
$R^6$ et $R^7$ sont égaux ou différents et représentent de l'hydrogène, un radical alkyle linéaire ou ramifié ayant jusqu'à 4 atomes de carbone ou un radical benzyle,
$R^5$ représente un reste de formule

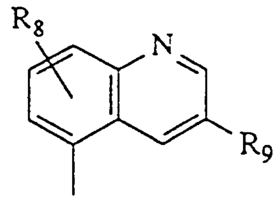 ou 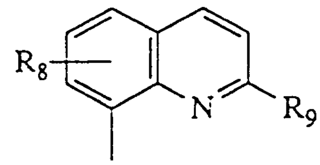

dans laquelle

$R^8$ est de l'hydrogène, un halogène ou un groupe alkyle ou alkoxy linéaire ou ramifié ayant chacun jusqu'à 4 atomes de carbone,

$R^9$ est un groupe phényle qui est substitué le cas échéant jusqu'à deux fois identiques ou différentes par un halogène, un groupe nitro, cyano, trifluorométhyle ou par un groupe alkyle ou alkoxy linéaire ou ramifié ayant chacun jusqu'à 6 atomes de carbone, par un groupe benzyle ou par un groupe de formule $-NR^6R^7$,

où

$R^6$ et $R^7$ ont la définition indiquée ci-dessus,

ou bien

représente un groupe 2-thiényle ou 3-thiényle qui est éventuellement substitué par un halogène,

et leurs sels acceptables du point de vue physiologique.

**3.** Composés de formule générale (I) suivant la revendication 1, dans laquelle

$R^1$ et $R^2$ sont égaux ou différents et représentent de l'hydrogène, un groupe méthyle, éthyle, propyle ou isopropyle, ou bien l'un des deux substituants $R^1$ et $R^2$ est un groupe $NH^2$,

X est un atome d'oxygène ou le groupe -NH,

$R^3$ et $R^4$ sont égaux ou différents et représentent de l'hydrogène, un groupe cyclopropyle, cyclopentyle ou un groupe alkyle linéaire ou ramifié ayant jusqu'à 6 atomes de carbone, qui est substitué le cas échéant par un radical carboxy, fluoro, chloro, cyano, hydroxy ou par un radical alkoxy, acyle ou alkoxycarbonyle linéaire ou ramifié ayant chacun jusqu'à 4 atomes de carbone ou par un groupe de formule $-NR^6R^7$,

où

$R^6$ et $R^7$ sont égaux ou différents et représentent de l'hydrogène, un radical méthyle, éthyle ou benzyle,

$R^5$ est un groupe de formule

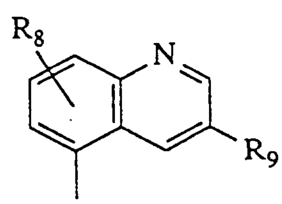 ou 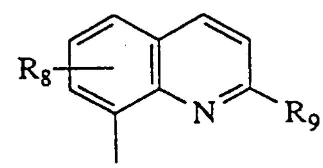

dans laquelle

$R^8$ est de l'hydrogène, un groupe éthyle ou méthyle,

$R^9$ est un groupe phényle qui est substitué le cas échéant jusqu'à deux fois identiques ou différentes par du fluor, du chlore, un radical nitro, trifluorométhyle, ou par un radical alkyle ou alkoxy linéaire ou ramifié ayant chacun jusqu'à 4 atomes de carbone ou par un groupe de formule $-NR^6R^7$,

où

$R^6$ et $R^7$ ont la définition indiquée ci-dessus,

ou

représente un groupe 2- ou 3-thiényle,

et leurs sels acceptables du point de vue physiologique.

**4.** Composés de formule générale (I) suivant la revendication 1, destinés à être utilisés pour combattre des maladies du système circulatoire.

**5.** Procédé de production de composés de formule générale (I)

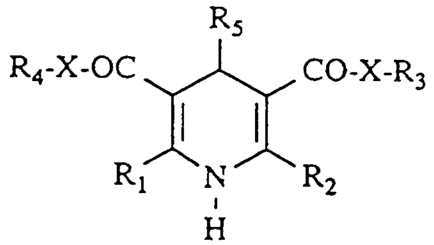

(I)

dans laquelle

$R^1$ et $R^2$ sont égaux ou différents et représentent de l'hydrogène ou un groupe alkyle linéaire ou ramifié ayant jusqu'à 6 atomes de carbone,

ou bien

l'un des deux substituants $R^1$ et $R^2$ est un groupe $-NH_2$,

X est un atome d'oxygène ou le groupe -NH,

$R^3$ et $R^4$ sont égaux ou différents et représentent de l'hydrogène ou un groupe cycloalkyle ayant 3 à 6 atomes de carbone, ou bien un groupe alcényle ou un groupe alkyle linéaire ou ramifié ayant chacun jusqu'à 10 atomes de carbone, dont la chaîne est interrompue le cas échéant par un atome d'oxygène ou de soufre et/ou qui sont substitués le cas échéant par un radical carboxy, halogéno, cyano, hydroxy, phényle, phénoxy, benzyloxy ou par un radical alkoxy, acyle ou alkoxycarbonyle linéaire ou ramifié ayant jusqu'à 8 atomes de carbone ou par un groupe de formule $-NR^6R^7$,

où

$R^6$ et $R^7$ sont égaux ou différents et représentent de l'hydrogène, un groupe alkyle linéaire ou ramifié ayant jusqu'à!6 atomes de carbone, un groupe benzyle ou phényle,

$R^5$ est un reste de formule

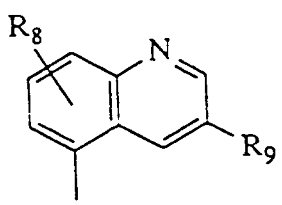

où

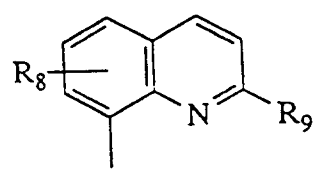

où

$R^8$ représente de l'hydrogène, un halogène ou un groupe alkyle ou alkoxy linéaire ou ramifié ayant jusqu'à 6 atomes de carbone,

$R^9$ est un groupe aryle ayant 6 à 10 atomes de carbone, qui peut être substitué le cas échéant jusqu'à 3 trois identiques ou différentes par un radical halogéno, nitro, cyano, trifluorométhyle, trifluorométhoxy, trifluorométhylthio ou par un groupe alkyle linéaire ou ramifié ayant jusqu'à 8 atomes de carbone, qui peut être substitué quant à lui par un radical aryle ayant jusqu'à 10 atomes de carbone,

ou bien par un radical alkoxy ou alkoxycarbonyle linéaire ou ramifié ayant chacun jusqu'à 8 atomes de carbone, un radical carboxy ou un groupe de formule $-NR^6R^7$,

où

$R^6$ et $R^7$ ont la définition indiquée ci-dessus,

ou représente un groupe 2- ou 3-thiényle qui est éventuellement substitué par un halogène

et de leurs sels acceptables du point de vue physiologique, caractérisé en ce que

[A] on fait réagir des aldéhydes de formule générale (II)

$R^5$-CHO (II)

dans laquelle

$R^5$ a la définition indiquée ci-dessus, tout d'abord avec des dérivés d'acide acétique de formule générale (III)

$R^{2'}$-CO-$CH_2$-CO-X-$R^3$ (III)

dans laquelle
$R^3$ et X ont la définition indiquée ci-dessus, et $R^{2'}$ a la définition indiquée ci-dessus pour $R^2$, mais ne représente pas un groupe amino,
le cas échéant avec isolement du composé ylidénique correspondant de formule générale (IV)

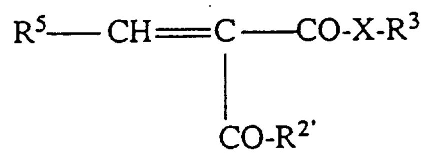

(IV)

dans laquelle
$R^{2'}$, $R^3$, $R^5$ et X ont la définition indiquée ci-dessus,
puis on fait réagir le produit avec des composés de formule générale (V)

$R^{1'}$-CO-$CH_2$-CO-X-$R^4$ (V)

dans laquelle
$R^4$ et X ont la définition indiquée ci-dessus et $R^{1'}$ ont la définition indiquée ci-dessus pour $R^1$, mais ne représente pas un groupe amino,
en présence d'ammoniac ou de sels d'ammonium ou directement avec des énamines de formule générale (VI)

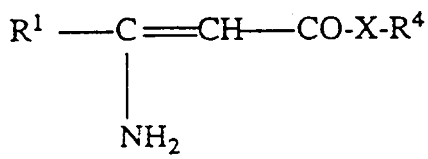

(VI)

dans laquelle
$R^1$, $R^4$ et X ont la définition indiquée ci-dessus,
le cas échéant en présence de solvants organiques inertes,
ou bien
[B] on fait réagir les aldéhydes de formule générale (II) tout d'abord avec des composés de formule générale (V), le cas échéant avec isolement des composés ylidéniques de formule générale (VII)

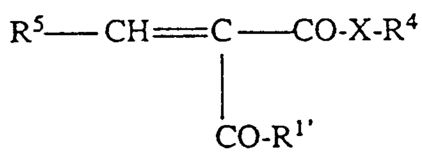

(VII)

dans laquelle
$R^{1'}$, $R^4$, $R^5$ et X ont la définition indiquée ci-dessus, et dans une étape ultérieure, on fait réagir le produit avec des composés de formule générale (III) dans des solvants inertes, en présence d'ammoniac ou de sels d'ammonium ou directement avec les dérivés d'énaminoacides carboxyliques de formule générale (VIII)

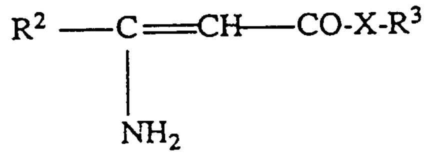

(VIII)

dans laquelle
$R^2$, $R^3$ et X ont la définition indiquée ci-dessus,
ou bien
[C] au cas où $R^4$ ne représente pas de l'hydrogène, on fait réagir des composés de formule générale (I) dans laquelle $R^1$, $R^2$ et $R^5$ ont la définition indiquée ci-dessus et $R^3$ représente de l'hydrogène, le cas échéant en passant par un dérivé d'acide réactif, avec les alcools ou les amines correspondants, et on obtient par addition des dérivés de pureté énantiomérique ($R^3$ = H) des énantiomères correspondants des composés de formule (I).

6. Médicament contenant au moins un composé de formule générale (I) suivant la revendication 1.

7. Procédé de préparation de médicaments, caractérisé en ce que des composés de formule générale (I) suivant la revendication 1 sont mis sous une forme administrable appropriée, éventuellement avec des substances auxiliaires et des supports classiques.

8. Utilisation de composés de formule générale (I) suivant la revendication 1 pour l'obtention de médicaments destinés au traitement de troubles du système circulatoire et de l'équilibre des liquides.